# EUROPÄISCHE PATENTSCHRIFT

(11) **EP 1 189 064 B1**
(45) Veröffentlichungstag und Bekanntmachung des Hinweises auf die Patenterteilung: **19.03.2003**
(21) Anmeldenummer: 01119090.7
(22) Anmeldetag: 08.08.2001
(51) Int. Cl.: G01N 33/96, G01N 33/52

(54) **Verfahren zum Kontrollieren der Gebrauchstauglichkeit von Analyseelementen**
Method for controlling the useability of analytical elements
Méthode pour vérifier l'utilisabilité d'éléments d'essai

(30) Priorität: 01.09.2000 DE 10043556
(43) Veröffentlichungstag der Anmeldung: 20.03.2002
(73) Patentinhaber: Roche Diagnostics GmbH, 68305 Mannheim (DE); F. HOFFMANN-LA ROCHE AG, 4070 Basel (CH)
(72) Erfinder: Gaa, Otto, 67551 Worms (DE); Albrecht, Gertrud, 68239 Mannheim (DE); Loch-Leroux, Dieter, 67316 Carlsberg (DE)
(74) Vertreter: Jany, Peter, Dr.

(56) Entgegenhaltungen:
- EP-A- 0 305 563
- DE-A- 2 800 225
- US-A- 4 436 812

## Beschreibung

Die Erfindung betrifft ein Verfahren zum Kontrollieren der Gebrauchstauglichkeit von Analyseelementen, bei dem kontrolliert wird, ob ein für mindestens einen Kontrollparameter eines kontrollierten Analyseelements gemessener Kontrollwert innerhalb eines Toleranzbereichs liegt.

Zur qualitativen und quantitativen Analyse mittels chemischer und biochemischer Verfahren für die Analyse von Bestandteilen von Probenmaterialien, insbesondere flüssiger Proben, insbesondere einer Körperflüssigkeit von Menschen oder Tieren, werden in großem Umfang in darauf spezialisierten Labors und insbesondere auch für den Einsatz außerhalb fester Labors sogenannte trägergebundene Schnelltests eingesetzt. Solche trägergebundenen Schnelltests basieren auf einer eigens entwickelten Trockenchemie und sind trotz der oftmals komplexen Reaktion unter Beteiligung empfindlicher Reagenzien selbst von Laien einfach und unkompliziert durchzuführen.

Dabei werden Analyseelemente verwendet, bei denen Reagenzien in eine oder mehrere Testschichten eingebettet sind. Zur Durchführung einer Reaktion wird das Analyseelement mit der Probe in Kontakt gebracht. Die Reaktion von Probe und Reagenz führt zu einer mit Hilfe eine Auswertegeräts (meistens reflektionsfotometrisch) oder visuell auswertbaren, für die Analyse charakteristischen Veränderung des Analyseelements. Die Analyseelemente werden auch als Testelemente bezeichnet.

Es sind zahlreiche unterschiedliche Analyseelement-Typen bekannt, die sich durch das Meßprinzip (z.B. optisch oder elektrochemisch) und die verwendeten Reagenzien sowiedurch ihren Aufbau, insbesondere durch die Anordnung und Befestigung der Testschichten, unterscheiden. Gebräuchlich sind insbesondere streifenförmige Analyseelemente (Teststreifen), die aus einem länglichen Kunststoffstreifen ("Basisstreifen") und mindestens einer daran befestigten Testschicht bestehen. Bei einem anderen gebräuchlichen Analyseelementtyp wird ein Kunststoffrahmen verwendet, der mindestens eine Testschicht einrahmt.

Ein bekanntes Beispiel für trägergebundene Schnelltests sind Testelemente für die Bestimmung des Blutglukosegehalts bei Diabetikern. Andere bekannte Ausführungsformen streifenförmiger diagnostischer Testelemente sind z.B. Ein- oder Meerfeldteststreifen für die Urinanalytik und diverse Indikatorpapiere. Apparativ auswertbare Analyseelemente, beispielsweise elektrochemisch oder optisch, insbesondere fotometrisch auswertbare Analyseelemente sind im Stand der Technik umfassend beschrieben und dem Fachmann in einer Vielzahl von Ausführungsformen geläufig.

Es ist bekannt, daß die Analyseelemente durch Umwelteinflüsse wie Licht, Temperatur oder Feuchte in ihrer analytischen Funktion geschädigt werden können. Derartige Schädigungen können bei der Anwendung zu Meßfehlern, die gefährliche Folgen für eine darauf basierende Diagnose haben können, führen oder die Analyseelemente unbrauchbar machen.

Fotometrisch ausgewertete Analyseelemente können beim Vorhandensein licht-, temperatur- oder feuchteempfindlicher Verbindungen bereits vor der Verwendung in einer Analyse ihre Trockenfärbung so stark verändern, daß dies von einem aufmerksamen Benutzer erkannt werden kann. In diesen Fällen können die Analyseelemente manuell ausgesondert werden. Die visuelle Beurteilung ist allerdings schwierig und setzt Erfahrung voraus. Darüber hinaus wird sie wegen der methodischen Schwierigkeiten zumeist nicht angewendet oder, insbesondere von Laien, einfach vergessen.

Um die Analyseelemente vor der Einwirkung von Lichtstrahlen, dem Zutritt von Luftfeuchtigkeit, Schmutz, Keimen, Staub sowie vor mechanischer Beeinträchtigung zu schützen oder unter sterilen Bedingungen aufzubewahren werden die Analyseelemente in der Regel verpackt. Dabei können die Analyseelemente entweder einzeln verpackt sein oder in einer gemeinsamen Packung oder einem Vorratsbehältnis zusammengefaßt sein. Die Verpackung bietet jedoch keinen 100%-igen Schutz. Sie kann beispielsweise bei der Herstellung, beim Transport oder bei einer unsachgemäßen Lagerung beschädigt werden. Von besonderer Bedeutung ist jedoch, daß sie nur einen sehr geringen Schutz gegen Temperatureinflüsse bietet und kaum oder keinen Schutz gegen Alterungseffekte gewährleistet. Der Alterungsprozeß erfolgt nach dem Öffnen einer Packung oder eines Vorratsbehältnisses verstärkt, so daß insbesondere die Alterung von Analyseelementen in diesem Zeitraum erfaßt werden sollte.

Zur Kontrolle der Gebrauchstauglichkeit eines Analyseelementes ist es daher bei fotometrischen Meßsystemen im Stand der Technik bekannt, vor dem Auftrag der zu messenden Probe den sogenannten Trockenleerwert des Meßfelds, d.h. den Remissionswert des Analyseelements ohne aufgetragene Probe zu bestimmen. Dieser Trockenleerwert wird gegen einen Vergleichswert abgeglichen und das Analyseelement wird zurückgewiesen, wenn der als Kontrollparameter dienende Trockenleerwert nicht innerhalb eines vorgegebenen Toleranzbereichs liegt.

Bei diesem bekannten Verfahren ist jedoch nur eine sehr grobe Überprüfung möglich, da die zur Auswertung der Analyseelemente verwendeten Meßgeräte bei der Fertigung individuelle Toleranzen aufweisen und die Geräteunterschiede sich während ihrer Lebensdauer im Gebrauch noch verstärken, beispielsweise durch Veränderungen an der Meßeinrichtung, insbesondere wenn es sich dabei um ein optisches Meßsystem handelt. Derartige Veränderungen können beispielsweise durch mechanische Belastungen, die zur Dejustierung führen, eine Alterung der Beleuchtungssysteme (beispielsweise von Lumineszensdioden) oder Staubablagerungen auf den optischen Elementen verursacht sein. Darüber hinaus haben auch den Trockenleerwert beeinflussende Chargenschwankungen zur Folge, daß nur eine grobe Überprüfung möglich ist, es sei denn, daß diese Chargenschwankungen durch chargenspezifische Informationen, die von den Analyseelementen oder ihrer Verpackung in das Auswertegerät übernommen werden, Berücksichtigung finden.

Bei elektrochemischen Analyseelementen ist, im Gegensatz zu fotometrischen Analyseelementen, bisher keine Möglichkeit zur Kontrolle oder Überwachung der Gebrauchstauglichkeit bekannt.

Aus dem Dokument US 4,832,488 ist es bekannt, die Alterungseffekte von optischen Analyseelementen, die zu einer Veränderung der in die Analyse eingehenden Kalibrationskurve führen, durch den Alterungsprozeß beschreibende Parameter zu berücksichtigen. Die Parameter werden anhand optischer Messungen an Analyseelementen, zu denen in manchen Fallkonstellationen auch die Messung des Trockenleerwerts gehört, ermittelt und die anhand dieser Parameter berichtigte Kalibrationskurve führt zu einer besseren Meßgenauigkeit der Analyse. Dieses Verfahren ist in der praktischen Anwendung jedoch aufwendig und beinhaltet keine Kontrolle der grundsätzlichen Gebrauchstauglichkeit der Analyseelemente.

Aus dem Dokument DE 2800225 A1 sind Teststreifenelemente bekannt, die ein Referenzfeld mit Vergleichs- oder Kompensationspapierstücken aufweisen. Diese werden, ebenso wie das eigentliche Testfeld, mit verschiedenen Wellenlängen bestrahlt und das Reflexionsvermögen gemessen. Es werden die durch Proben auf dem Referenzfeld verursachten Veränderungen ermittelt und damit wird die durch Eigenschaften der Probe bedingte Beeinflussung des Meßfeldes, die nicht auf der Konzentration des Analyten beruht, kompensiert. Es handelt sich also um eine Korrektur von probenbedingten Veränderungen der Meßbedingungen an einem Analysegerät. Eine Kontrolle der Gebrauchstauglichkeit eines Analyseelements erfolgt nicht.

Der Erfindung liegt unter Berücksichtigung dieses Standes der Technik die Aufgabe zugrunde, ein einfaches und zuverlässiges Verfahren zum Kontrollieren der Gebrauchstauglichkeit von Analyseelementen zu schaffen.

Diese Aufgabe wird erfindungsgemäß durch ein Verfahren mit den Merkmalen des beigefügten Anspruchs 1 gelöst. Bevorzugte Ausgestaltungen und Weiterbildungen der Erfindung ergeben sich aus den Ansprüchen 2 bis 18 und der nachfolgenden Beschreibung mit zugehörigen Zeichnungen.

Ein erfindungsgemäßes Verfahren zum Kontrollieren der Gebrauchstauglichkeit von Analyseelementen, bei dem kontrolliert wird, ob ein für mindestens einen Kontrollparameter eines kontrollierten Analyseelements gemessener Kontrollwert innerhalb eines Toleranzbereichs liegt, umfaßt also folgende Schritte:
a) In einem ersten Schritt wird in einer Referenzwertmessung ein erster Standardreferenzwert an einem Referenzkontrollmittel bestimmt. Das Referenzkontrollmittel stellt einen standardisierten Referenzwert für den Kontrollparameter bereit.
b) In einem zweiten Schritt wird als Kontrollbezugswert der Kontrollparameter eines ersten Analyseelements bestimmt.
c) In einem dritten Schritt wird der Quotient aus dem Kontrollbezugswert und dem ersten Standardreferenzwert gebildet und für später gemessene Analyseelemente als erster Bezugsquotient gesetzt.
d) In einem vierten Schritt wird zur Kontrolle eines zu kontrollierenden zweiten Analyseelements der Kontrollparameter des zweiten Analyseelements als Kontrollwert bestimmt.
e) In einem fünften Schritt wird als Kontrollquotient der Quotient aus dem Kontrollwert und dem ersten Standardreferenzwert gebildet.
f) In einem sechsten Schritt wird die Abweichung zwischen dem Kontrollquotienten und dem ersten Bezugsquotienten bestimmt.
g) Das kontrollierte zweite Analyseelement wird zurückgewiesen, wenn die Abweichung außerhalb eines vorgegebenen Toleranzbereichs liegt.

Die Erfindung wird im folgenden ohne Beschränkung der Allgemeinheit am Beispiel fotometrisch ausgewerteter Analyseelemente beschrieben. Die erläuterten Prinzipien lassen sich ohne weiteres auf andere, beispielsweise elektrochemische Analyseelemente übertragen.

Bei elektrochemischen Analyseelementen kann eine zur Durchführung der Kontrolle geeignete elektronische Meßgröße verwendet werden. In Betracht kommt beispielsweise eine Leerwertmessung mit einer Wechselspannung, bei der die Impedanz oder Phasenverschiebung den Kontrollparameter liefert. Andere mögliche Kontrollparameter sind Leitfähigkeit, Kapazität, Induktivität, Abklingzeiten, Frequenz- oder in Kennlinien ausdrückbare funktionale Zusammenhänge von elektronischen Meßgrößen, z.B. eine strom-, spannungs- oder frequenzabhängige Charakteristik. In Fällen, in denen das eigentliche elektrochemische Analyseelement selbst keinen geeigneten Kontrollparameter aufweist, ist es möglich, zur meßtechnischen Ermöglichung einer Kontrolle dem Analyseelement eine zusätzliche Komponente hinzuzufügen, die ein meßtechnisch erfaßbares und für die erfindungsgemäßen Kontrollzwecke geeignetes Merkmal bereitstellt.

Das erfindungsgemäße Verfahren umfaßt somit im wesentlichen zwei Aspekte. Gemäß dem ersten Aspekt werden Änderungen der Meßoptik überwacht. Hierzu wird ein Referenzwert an einer standardisierten Referenzfläche, z.B. eine Weißstandardmessung, durchgeführt, die einen standardisierten Referenzwert für den Kontrollparameter, bereitstellt. Auf die Referenzfläche wird kein Probenmaterial aufgegeben. Ein im erfindungsgemäßen Rahmen besonders geeigneter Kontrollparameter ist ein optischer Meßwert, insbesondere ein.Remissionswert. Der auf diese Weise bestimmte erste Standardreferenzwert überprüft den aktuellen Zustand des Auswertegeräts, beispielsweise der Meßoptik, und kann zur Nachkalibration des Auswertegeräts herangezogen werden; dies ist im erfindungsgemäßen Zusammenhang jedoch nicht erforderlich, und es reicht aus, wenn der Standardreferenzwert als Bezugsgröße für nachfolgende Messungen verwendet wird.

Der zweite Aspekt richtet sich auf die Kontrolle der Gebrauchstauglichkeit einzelner Analyseelemente, die beispielsweise durch Alterung oder schädigende Einflüsse beeinträchtigt sein kann. Hierzu wird der Kontrollparameter eines ersten Analyseelements an dem reaktiven Meßfeld vor dem Aufbringen des Probenmaterials bestimmt und als Kontrollbezugswert für später gemessene Analyseelemente herangezogen. Zu diesem Zweck wird der Quotient aus dem Kontrollbezugswert des ersten Analyseelements und dem ersten Standardreferenzwert gebildet und der Quotient als erster Bezugsquotient gesetzt. Bei der nachfolgenden Kontrolle eines auf seine Gebrauchstauglichkeit zu kontrollierenden zweiten Analyseelements wird an dem reaktiven Meßfeld vor dem Aufbringen des Probenmaterials der Kontrollparameter, d.h. beispielsweise der Trockenleerwert des zweiten Analyseelements als Kontrollwert bestimmt und als Kontrollquotient der Quotient aus dem bestimmten Kontrollwert des zweiten Analyseelements und dem ersten Standardreferenzwert gebildet. Anhand der Abweichung zwischen dem Kontrollquotienten und dem ersten Bezugsquotienten kann dann geprüft werden, ob das kontrollierte zweite Analyseelement gebrauchsfähig ist oder, wenn die Abweichung außerhalb eines vorgegebenen Toleranzbereichs liegt, wegen einer Schädigung zurückzuweisen ist.

Mit dem erfindungsgemäßen Verfahren ist es möglich, ohne aufwendige Nachkalibrationsvorgänge vor der Durchführung einer Analyse einfach und zuverlässig zu kontrollieren, ob ein Analyseelement zur Durchführung einer Analyse verwendet werden kann oder wegen Vorliegens einer Schädigung, insbesondere einer Alterung nach Packungsanbruch, zurückgewiesen werden muß.

Bei dem Verfahren geht man davon aus, daß das erste Analyseelement, mit dem der Kontrollbezugswert bestimmt wird, unbeschädigt und gebrauchsfähig ist. Die Gebrauchsfähigkeit des ersten Analyseelements wird nicht mittels des erfindungsgemäßen Verfahrens kontrolliert. Dies kann jedoch nach dem im Stand der Technik bekannten Verfahren zumindest in grober Weise anhand des Trockenleerwerts des ersten Analyseelements überprüft werden; wenn der an dem ersten Analyseelement ermittelte Kontrollbezugswert nicht innerhalb einer vorgegebenen Toleranz liegt, ist das erste Analyseelement nicht gebrauchstauglich.

Das erfindungsgemäße Verfahren funktioniert prinzipiell auch ohne die Referenzmessung im Schritt a). Durch den Schritt a) wird jedoch das Verfahren sensitiver, da hierdurch die Unterschiede verschiedener Analysegeräte, beispielsweise bauartbedingte Streuungen in den optischen Meßeinrichtungen, berücksichtigt werden. Die grobe Kontrolle des ersten Analyseelements ist jedoch nicht Gegenstand der Erfindung, die sich auf die Kontrolle der auf das erste Analyseelement folgenden weiteren Analyseelemente richtet und hierzu das oben erläuterte Verfahren vorschlägt.

Das erfindungsgemäße Verfahren ist daher gemäß einem zusätzlichen Aspekt insbesondere für solche Anwendungsfälle vorteilhaft, bei denen das erste Analyseelement und das zweite, zu kontrollierende Analyseelement Teil einer Packung oder eines Vorratsbehältnisses sind, die weitere gleichartige Analyseelemente enthält und eine den Analyseelementen gemeinsame Langzeitverpackung aufweist. Dabei kann nach einem zusätzlichen vorteilhaften Merkmal vorgesehen sein, daß die Analyseelemente in der Packung jeweils durch ein individuelles Verpackungselement zusätzlich einzeln geschützt sind.

In diesen Anwendungsfällen wird durch die gemeinsame Langzeitverpackung mit einem relativ hohen Grad an Sicherheit gewährleistet, daß die darin enthaltenen Analyseelemente und somit das erste Analyseelement nicht geschädigt sind, ausgenommen ggf. Schädigungen durch Temperatureinflüsse, die über die bekannte Leerwerttoleranzmessung erfaßt werden können. Nach dem Öffnen der Langzeitverpackung ist die Gefahr, daß durch eine unsachgemäße Handhabung einzelne in der Packung enthaltene Analyseelemente geschädigt werden, hoch. Eine Beeinträchtigung der Gebrauchsfähigkeit einzelner Analyseelemente nach dem Öffnen der Langzeitverpackung kann jedoch vorteilhafterweise durch die erfindungsgemäße Kontrolle erkannt werden.

Die Erfindung hat den Vorteil, daß eine einfache und zuverlässige Kontrolle von Analyseelementen ermöglicht wird. Ein zusätzliches vorteilhaftes Merkmal besteht darin, daß einer oder mehrere der Verfahrensschritte automatisch, d.h. benutzerunabhängig durchgeführt werden. Es wird angemerkt, daß die Reihenfolge der erfindungsgemäßen Schritte in zweckmäßiger Weise abgeändert werden kann - beispielsweise kann die Reihenfolge der Schritte a) und b) vertauscht werden - und die Bezeichnungen "erster" Schritt usw. insoweit nur zur Differenzierung der einzelnen Verfahrensschritte ohne Festlegung der Reihenfolge dienen; die in Anspruch 1 angegebene Reihenfolge der Verfahrensschritte stellt jedoch eine bevorzugte Ausführungsform dar.

Desgleichen bezeichnen die Begriffe "erstes Analyseelement" und "zweites Analyseelement" nicht einschränkend das erste gemessene Analyseelement (einer Packung) bzw. das unmittelbar folgende Analyseelement, sondern bezeichnen nur eine begriffliche Differenzierung. Das erste Analyseelement ist dasjenige, mit dem der Standardreferenzwert bestimmt wird. Dies ist vorzugsweise aber nicht notwendigerweise das erste Analyseelement einer Packung. Das zweite Analyseelement ist eines der nachfolgend bzw. relativ zu dem ersten Analyseelement kontrollierten Analyseelemente. Dies sind vorzugsweise aber nicht notwendigerweise alle auf das erste Analyseelement folgenden Analyseelemente.

Die Erfindung wird nachfolgend anhand eines in den Figuren dargestellten Ausführungsbeispiels näher erläutert.

Die beschriebenen Besonderheiten können einzeln oder in Kombination miteinander eingesetzt werden, um bevorzugte Ausgestaltungen der Erfindung zu schaffen. Es zeigen:
- Fig. 1: ein Analyseelement in Form eines Teststreifens und
- Fig. 2: eine Packung mit Teststreifen, die in einem Vorratsbehältnis untergebracht sind.

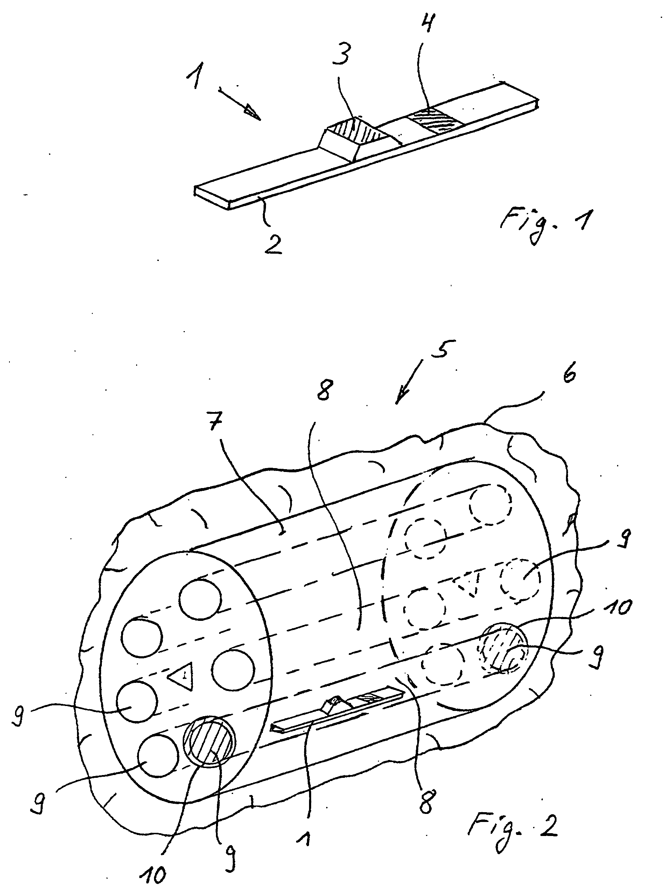

Die Fig. 1 zeigt ein Analyseelement 1 in Form eines Teststreifens, der in ein nicht dargestelltes Auswertegerät eingeführt werden kann; das Auswertegerät und die darauf abgestimmten Analyseelemente 1 bilden ein Analysesystem. Das Analyseelement weist eine elastische Basisschicht 2 auf, die üblicherweise aus einem Kunststoffmaterial besteht. Sie trägt ein Testfeld 3, auf die eine zu analysierende Probenflüssigkeit aufgetragen wird. Dabei finden chemische Reaktionen zwischen der Probenflüssigkeit und den in dem Testfeld 3 enthaltenen Reagenzien statt. Eine resultierende, optisch nachweisbare Veränderung kann reflektionsfotometrisch detektiert werden.

Es sind viele Ausführungsformen von Testelementen bekannt, die sich beispielsweise durch die Art der chemischen Nachweisreaktion, den Schichtenaufbau in dem Testfeld, die Methode der optischen Messung und die Anzahl der Testfelder 3 auf einem Teststreifen unterscheiden. Alle vorbekannten Teststreifen sind im Rahmen des erfindungsgemäßen Kontrollverfahrens geeignet.

Die Gebrauchsfähigkeit des Analyseelements 1 kann anhand einer sogenannten Trockenleerwertmessung des Testfelds 3, d.h. einer optischen Messung des noch nicht mit Probenflüssigkeit benetzten Testfelds 3 grob überprüft werden. Hierzu wird das Analyseelement 1 vor der Benetzung mit der Probenflüssigkeit in das Auswertegerät eingeschoben und der Trockenleerwert gemessen. Liegt der als Kontrollparameter dienende Trockenleerwert innerhalb eines vorgegebenen Toleranzbereichs, der ggf. chargenspezifisch festgelegt wird, so besteht eine erhöhte Wahrscheinlichkeit dafür, daß das Analyseelement 1 nicht geschädigt und somit gebrauchsfähig ist. Es wird dann aus dem Auswertegerät entnommen, mit der Probenflüssigkeit benetzt und zur Durchführung der eigentlichen Analyse wieder in die Meßeinrichtung des Auswertegeräts eingeführt. Das Auswertegerät kann aber zweckmäßigerweise auch so ausgebildet sein, daß ein Probenauftrag ohne nochmalige Entnahme des Analyseelements 1 möglich ist.

Zur Durchführung des erfindungsgemäßen Kontrollverfahrens weist das Analyseelement 1 neben dem zur Kontrolle seiner Gebrauchstauglichkeit und der Durchführung der Analyse dienenden Testfeld 3 vorteilhafterweise zusätzlich ein integriertes Referenzkontrollmittel auf, das einen standardisierten Referenzwert für den Kontrollparameter, beispielsweise den Remissionswert bereitstellt. Das Referenzkontrollmittel erfährt keine Veränderung durch das Probenmaterial; es ist also unreaktiv, dient nicht zum Aufsaugen von Probenmaterial und es haftet kein Probenmaterial an ihm an. Das Referenkontrollmittel ist in Form eines Referenzfeldes 4 ausgebildet, das einen standardisierten Referenzwert für die Remission liefert. Es handelt sich dabei in einer bevorzugten Ausführungsform beispielsweise um ein Referenzkontrollmittel, das aus einer weißen Kunststoffolie besteht oder eine solche beinhaltet.

Der Remissionswert des Referenzfeldes ist vorzugsweise hoch, z.B. 80 %, um einen hohen Meßwert für den Kontrollparameter Remission und somit eine relativ hohe Meßgenauigkeit zu ermöglichen. Im Rahmen der Erfindung werden jedoch keine sehr hohen Anforderungen an das als Referenzkontrollmittel dienende Referenzfeld 4 gestellt. So kommt beispielsweise auch eine weniger stark remittierende graue Fläche in Betracht und der Remissionsgrad des Referenzfeldes 4 muß nicht langfristig stabil sein. Es ist jedoch bevorzugt, wenn die Eigenschaften des Referenzfeldes 4 weitgehend unabhängig von den Umgebungsbedingungen wie Temperatur und Feuchte sind.

In Fig. 1 ist das Referenzfeld 4 als speziell ausgebildeter Teil des Analyseelements 1 dargestellt. Aufgrund der vorstehend erläuterten unkritischen Anforderungen an die Eigenschaften des Referenzfeldes 4 ist es aber auch möglich, einen nicht speziell ausgebildeten Abschnitt des Analyseelements 1 als Referenzkontrollmittel heranzuziehen, z.B. einen Teil der Oberfläche des vorzugsweise weißen Kunststoffs, aus dem ein Teststreifen bzw. dessen Basisschicht 2 besteht. Die Markierung des Referenzfeldes 4 in Fig. 1 veranschaulicht in diesen Fällen nur, welche Stelle des Analyseelements 1 als Referenzkontrollmittel dient, ohne daß dieser Bereich sich von dem benachbarten Material unterscheidet.

Das Referenzfeld 4 kann wie in Fig. 1 dargestellt integrierter Bestandteil des Analyseelements 1 sein. Es ist aber auch möglich, das Referenzfeld 4 auf einem separaten, ggf. ohne Testfeld 3 versehenen Referenzteststreifen auszubilden, der dem Auswertegerät und/oder einer Packung bzw. Charge von Analyseelementen 1 beigegeben wird. In anderen Ausführungsformen kann das Referenzfeld 4 bzw. allgemein das Referenzkontrollmittel ein integrierter Bestandteil des zugehörigen Auswertegeräts sein.

Das Referenzfeld 4 dient als Referenzgröße, mittels der der aktuelle Zustand des Auswertegeräts bzw. dessen Optik berücksichtigt werden kann. Mit ihm können apparativ bedingte Schwankungen der Meßbedingungen erfaßt werden. Hierzu wird in einem ersten Schritt in einer Referenzwertmessung ein erster Standardreferenzwert W1 eines Weißstandardwerts von beispielsweise 80 % Remission an dem Referenzfeld 4 gemessen. Hierzu wird das Analyseelement 1 in eine Position in dem Auswertegerät gebracht, in der nicht das Testfeld 3, sondern das Referenzfeld 4 gemessen wird.

Der Weißstandardwert W1 ergibt nur bei einer bestimmten Meßapparatur mit definierten und konstanten Meßeigenschaften einen festen, konstanten Wert. Dies läßt sich in der Praxis, insbesondere bei außerhalb von Labors befindlichen Meßapparaturen, nicht realisieren, so daß der Weißstandardwert W1 apparativen Schwankungen des Auswertegeräts (und ggf. Schwankungen hinsichtlich der Eigenschaften des Referenzfeldes 4) unterliegt. Dies ist im Rahmen der Erfindung jedoch nicht nachteilig, da gerade durch die Referenzierung auf einen Weißstandard im Wege der erfindungsgemäßen Quotientenbildung apparative Veränderungen, die sich auch kurzfristig ergeben können, berücksichtigt werden.

Danach oder ggf. auch davor wird in einem zweiten Schritt der Trockenleerwert L1 des Testfeldes 3, das hierzu in dem Auswertegerät in die Meßposition gebracht wird, bestimmt. Dabei kann optional auch gleichzeitig geprüft werden, ob der Trockenleerwert L1 innerhalb eines vorgegebenen, ggf. chargenabhängigen Toleranzbereichs liegt. In dem Ausführungsbeispiel wird angenommen, daß der Trockenleerwert gebrauchsfähiger Analyseelemente 1 zwischen 55 % und 65 % Remission und bei der konkreten Messung 60 % Remission beträgt.

In einem dritten Schritt wird aus dem an dem ersten Analyseelement gemessenen Kontrollbezugswert L1 und dem ersten Standardreferenzwert W1 ein Quotient gebildet und für später gemessene Analyseelemente als erster Bezugsquotient Q1 gesetzt. In dem angenommenen Beispiel beträgt der erste Bezugsquotient Q1 somit 60/80 = 0,75.

Das erste Analyseelement, anhand dessen der erste Bezugsquotient Q1 ermittelt wurde, kann daraufhin für die Durchführung einer Analyse verwendet werden. Hierzu wird es aus dem Auswertegerät entnommen, auf das Testfeld 3 die zu analysierende Probe aufgetragen und das Analyseelement 1 zur Auswertung in das Auswertegerät eingeführt, oder die Probe wird auf das in dem Auswertegerät belassene Analyseelement aufgebracht.

Bei Verwendung eines zweiten Analyseelements wird zunächst der Kontrollwert L2, d.h. der Trockenleerwert des Testfelds 3 dieses zweiten, zu kontrollierenden Analyseelements bestimmt. Aus diesem Trockenleerwert L2 wird durch Division durch den ersten Standardreferenzwert W1 der Kontrollquotient Q2 gebildet und danach die Abweichung Δ zwischen dem Kontrollquotienten Q2 und dem ersten Bezugsquotienten Q1 überprüft. Das kontrollierte zweite Analyseelement wird zurückgewiesen, wenn die Abweichung Δ außerhalb eines vorgegebenen Toleranzbereichs liegt.

Die Entscheidung, ob die Abweichung Δ außerhalb eines vorgegebenen Toleranzbereichs liegt oder nicht, kann beispielsweise anhand des Quotienten Q1/Q2, d.h. des relativen Unterschiedes zwischen dem ersten Bezugsquotienten Q1 und dem Kontrollquotienten Q2, anhand einer (absoluten) Differenz Q1-Q2 oder anhand einer funktionalen oder parametrischen Bewertung der Abweichung Δ getroffen werden.

Beträgt beispielsweise in dem angenommenen Beispiel der Trockenleerwert L2 56,4 % und der Toleranzbereich für die zulässige Abweichung Δ 6 % relativ, bezogen auf den ersten Bezugsquotienten Q1, oder 0,045 absolut, bezogen auf die Differenz von Q1 und Q2, so liegt der Kontrollquotient Q2 von 56,4/80 = 0,705 des kontrollierten zweiten Analyseelement gerade noch innerhalb des zuläs-sigen Bereichs und das zweite Analyseelement kann zur Verwendung in einer Analyse zugelassen werden. Ergibt dagegen die Messung des Kontrollwertes L2 einen Trocken-leerwert von 50 % für das zweite Analyseelement, so resultiert ein Kontrollquotient Q2 von 50/80 = 0,625 und eine Abweichung Δ von 16 % relativ bzw. 0,125 absolut. Das betreffende kontrollierte zweite Analyseelement wird dann nicht zur Verwendung in einer Analyse zugelassen und zurückgewiesen.

Der Toleranzbereich für die zulässige Abweichung Δ kann chargenspezifisch für die aktuelle Charge der Analyseelemente sein. Dies kann beispielsweise dadurch realisiert werden, daß die Analyseelemente oder deren Verpackung mit einem Strichcode versehen sind, der von dem Meßgerät ausgelesen und bei der Kontrolle oder Analyse berücksichtigt wird. Eine Abwandlung kann darin bestehen, daß in dem Auswertegerät ein Satz von Toleranzbereichen abgespeichert ist, aus denen über den eingelesenen Strichcode ein Wert für die spezielle Charge ausgewählt wird.

Die Messung des Standardreferenzwertes W1 könnte prinzipiell bei jedem Analyseelement erfolgen. Dies ist im Rahmen der Erfindung jedoch nicht erforderlich, so daß Zeit gespart wird, wenn die Messung nur im Bedarfsfall erfolgt.

Bei manchen Analyseelementen, insbesondere bei Testelementen für einen trägergebundenen Schnelltest zur qualitativen oder quantitativen Analyse von Bestandteilen einer festen oder flüssigen Probe, insbesondere einer Körperflüssigkeit von Menschen oder Tieren, insbesondere zur Bestimmung des Blutglukosegehalts kann vorgesehen sein, daß das kontrollierte, zweite Analyseelement in Schritt g) zurückgewiesen wird, wenn der Kontrollquotient Q2 um mehr als einen vorgegebenen Prozentsatz oder mehr als einen vorgegebenen Differenzbetrag kleiner als der erste Bezugsquotient Q1 ist. Im Rahmen der Erfindung hat sich nämlich herausgestellt, daß insbesondere bei den vorgenannten Testelementen Alterungs- und Schädigungseinflüsse zumeist zu einer Verringerung des Remissionswertes und damit einer Verringerung des Kontrollquotienten Q2 im Vergleich zum ersten Bezugsquotienten Q1 führen und daher die Abnahme zur Kontrolle herangezogen werden kann.

Nach einem anderen bevorzugten Merkmal, insbesondere bei den vorstehend genannten Testelementen, kann vorgesehen sein, daß beim Feststellen einer vorgegebenen Abweichung Δ in Schritt g) der in Schritt d) bestimmte Kontrollwert L2 als neuer Kontrollbezugswert gemäß Schritt b) verwendet, daraus ein neuer Bezugsquotient gemäß Schritt c) gebildet und der neue Bezugsquotient für die Kontrolle weiterer Analyseelemente gemäß den Schritten d) bis g) zugrundegelegt wird. Eine vorgegebene Abweichung Δ in diesem Sinn, die dazu führt, daß der später gemessene Kontrollquotient Q2 an die Stelle des ersten Bezugsquotienten Q1 tritt und diesen für folgende Kontrollen ersetzt, kann die Feststellung sein, daß der in Schritt e) bestimmte Kontrollquotient Q2 den aktuellen (ersten) Bezugsquotienten Q1 um mehr als einen festgelegten Grenzwert δ übersteigt.

In.dem oben genannten Beispiel führt beispielsweise eine Messung eines Kontrollwertes bzw. Trockenleerwertes L2 an einem zu kontrollierenden Analyseelement von größer als 61,8 % zu einem Kontrollquotienten Q2 von größer als 0,7725 und damit zum Ersetzen des ersten Bezugsquotienten Q1 von 0,75 durch den Kontrollquotienten Q2, wenn für den zulässigen Grenzwert δ ein Betrag von 3 %, bezogen auf den ersten Bezugsquotienten Q1, oder von 0,0225 als Abweichung von Q1 vorgegeben wird. In einer zusätzlichen vorteilhaften Ausführungsform kann vorgesehen sein, daß der Grenzwert δ chargenspezifisch für die aktuelle Charge der Analyseelemente ist.

Eine Vergrößerung des Kontrollquotienten Q2 im Vergleich zu dem ersten Bezugsquotienten Q1 kann sich beispielsweise dadurch ergeben, daß die Meßoptik des Auswertegeräts gereinigt wurde oder andere denkbare, die Kontrolle oder Analyse beeinflussende Maßnahmen durchgeführt wurden. Durch das erfindungsgemäße Verfahren kann dies erkannt und bei der Gebrauchsfähigkeitskontrolle der Analyseelemente berücksichtigt werden.

Es wird angemerkt, daß die beispielhaft erläuterten Quotienten und Differenzen auch umgekehrt, d.h. mit vertauschtem Dividenden und Divisor bzw. vertauschtem Minuenden und Subtrahenden gebildet werden können.

Das erfindungsgemäße Verfahren wird bevorzugt in Fällen angewendet, in denen das erste Analyseelement und das zweite, zu kontrollierende Analyseelement Teil einer Packung oder eines Vorratsbehältnisses sind, die weitere gleichartige Analyseelemente enthält und eine den Anlayseelementen gemeinsame Langzeitverpackung aufweist. In Fig. 2 ist schematisch eine solche Packung 5 dargestellt. Von den enthaltenen Analyseelementen 1 ist eines schematisch angedeutet. Die in einer Packung 5 zusammengefaßten Analyseelemente sind von einer Langzeitverpackung 6, die einen sicheren Schutz darstellt, umhüllt. Die Langzeitverpackung 6 kann beispielsweise eine hochfeste, verschweißte Folie oder ein röhrenförmiges Behältnis aus Kunststoff oder Metall, z.B. Aluminium sein.

In der Packung 5 können die mehreren darin enthaltenen Analyseelemente 1 nochmals jeweils durch ein individuelles Verpackungselement einzeln geschützt sein. Die Langzeitverpackung 6 gewährleistet dann eine sichere Aufbewahrung über längere Zeit bzw. einen sicheren Transport. Zur Verwendung der in der Packung 5 enthaltenen Analyseelemente 1 wird zunächst vom Endverbraucher.die Langzeitverpackung 6 geöffnet und die darin enthaltenen Analyseelemente 1 werden nach und nach verbraucht. In der Zeit zwischen dem öffnen der Langzeitverpackung 6 und der Verwendung eines bestimmten Analyseelements 1 daraus gewährleisten die individuellen Verpackungselemente den erforderlichen Mindestschutz. Mit dem erfindungsgemäßen Verfahren kann kontrolliert werden, ob dieser Mindestschutz tatsächlich erfüllt wurde oder ob alle oder einzelne Analyseelemente 1 seit dem Öffnen der Langzeitverpackung 6 in ihrer Gebrauchsfähigkeit geschädigt wurden.

Es ist auch bekannt, die Analyseelemente 1 in der Langzeitverpackung 6 nicht lose, sondern in einem Vorratsbehältnis 7 anzuordnen, das nicht nur eine Packmittelfunktion erfüllt, sondern auch ein Funktionsteil für die vorgesehene Verwendung der Analyseelemente 1 darstellen kann. Es ist auch möglich, daß mehrere Vorratsbehältnisse 7 in einer gemeinsamen Langzeitverpackung 6 untergebracht sind. Beispiele für derartige Vorratsbehältnisse und Entnahmeeinrichtungen finden sich in den Dokumenten DE 19854316 A1, EP 1022565 A2, US 4,615,462, US 4,834,234, US 5,645,798 und US 5,720,924.

In Fig. 2 ist beispielhaft ein solches Vorratsbehältnis 7 in Form eines Magazins dargestellt, in dessen Kammern 8 die Analyseelemente 1 angeordnet sind. Die Öffnungen 9 der Kammer 8 sind durch individuelle Verpackungselemente darstellende Siegelfolien 10 verschlossen, die zum Entnehmen eines Analyseelements 1 aus einer Kammer 8 des Vorratsbehältnisses 7 durchstoßen werden; dabei bleiben die Siegelfolien 10 der anderen, nicht entnommenen Analyseelemente 1 unverletzt und bilden einen Schutz für die in der jeweiligen Kammer 8 verbleibenden Analyseelemente 1.

Das als Magazin ausgebildete Vorratsbehältnis 7 ist für den Einsatz in einem Auswertegerät konzipiert. Für die Aufnahme eines Vorratsbehältnisses in ein Auswertegerät, in dem beispielsweise mit Hilfe eines Stößels ein Analyseelement aus einer Kammer entnommen wird, können entsprechende Mittel zum genauen Positionieren und Entnehmen vorgesehen sein.

Im Rahmen der Erfindung wird davon ausgegangen, daß bis zum Öffnen der Langzeitverpackung 6 die darin enthaltenen Analyseelemente 1 gebrauchsfähig sind oder eine evtl. Schädigung, beispielsweise durch eine sämtliche Analyseelemente betreffende Temperatureinwirkung nach einem konventionellen Kontrollverfahren ermittelt werden kann. Das Öffnen einer Langzeitverpackung 6 erfolgt in der Regel unmittelbar vor der Verwendung des ersten darin enthaltenen Analyseelements 1. Gemäß einer bevorzugten Ausführungsform der Erfindung wird daher vorgeschlagen, daß in Schritt b) das Analyseelement 1 gewählt wird, das als erstes einer Packung 5 oder einem in der Packung 5 enthaltenen Vorratsbehältnis 7 entnommen wird. Beispielsweise kann das Auswertegerät so ausgebildet sein, daß es automatisch beim Verwenden einer neuen Packung 5, beim Verwenden eines ersten Analyseelements 1 einer Packung 5 oder eines Vorratsbehältnisses 7 oder beim Öffnen einer Langzeitverpackung 6 die erfindungsgemäßen Schritte a), b) und c) durchführt.

Desweiteren kann vorteilhafterweise vorgesehen werden, daß die Schritte a) bis c) wiederholt werden, wenn an dem die Kontrollmessung bzw. die analytische Messung durchführenden Auswertegerät eine den Meßwert potentiell beeinflussende Änderung, beispielsweise ein Reinigen der Optik durchgeführt wurde. Eine Wiederholung der Schritte a) bis c) kann beispielsweise automatisch dann ausgelöst werden, wenn das Auswertegerät anhand eines Kontrollschalters registriert, daß eine Abdeckung des Auswertegerätes, die den Zugang zur Meßoptik ermöglicht, geöffnet wurde, da in diesem Fall möglicherweise eine Reinigung durchgeführt wurde.

In den nachfolgenden Tabellen 1 und 2 sind die Ergebnisse praktischer Erprobungen der Erfindung wiedergegeben. Dabei wurden Teststreifen überprüft, die zur Bestimmung des Blutglukosegehalts dienen. Die Teststreifen wurden bei 80% relative Luftfeuchte künstlich gealtert. Die hohe Luftfeuchte dient dabei als erhöhte Belastung, die die Alterungsvorgänge beschleunigt. Entsprechende Versuchsergebnisse wurden bei Untersuchungen mit erhöhter Temperatur oder unter realen Bedingungen mit verlängerten Alterungsdauern erzielt.

Die Tabelle 1 gibt die Belastungsergebnisse bei 80% relativer Luftfeuchte mit einem festen, ersten Standardreferenzwert W1 an. t ist die Dauer der Feuchtebelastung bei 80% relativer Feuchte in Stunden. Der in dem ersten Schritt a) gemessene Standardreferenzwert W1, der in dem zweiten Schritt b) gemessene Kontrollbezugswert L1 und der in dem vierten Schritt d) gemessene Kontrollwert L2 sind willkürlich in mV angegeben. In der Praxis können auch andere Einheiten auftreten, beispielsweise mA, Lichtstärke oder ein prozentualer Remissionswert. Der in dem dritten Schritt c) gebildete erste Bezugsquotient Q1 und der in dem fünften Schritt e) gebildete Kontrollquotient Q2 sind in Prozent angegeben. Die letzte Spalte der Tabelle zeigt die in dem sechsten Schritt f) bestimmte Abweichung Δ zwischen dem Kontrollquotienten Q2 und dem ersten Bezugsquotienten Q1; im dargestellten Beispielsfall ist die Differenz dieser Werte gewählt.

Man erkennt, daß mit zunehmender Belastungszeit die Alterungseffekte steigen und der Kontrollquotient Q2 abnimmt. Die Abweichung Δ zwischen dem Kontrollquotienten Q2 und dem ersten Bezugsquotienten Q1 nimmt dementsprechend zu. Wenn die bestimmte Abweichung Δ einen vorgegebenen Grenzwert überschreitet, kann auf diese Weise festgestellt werden, daß das zur Analyse vorgesehene Testelement nicht gebrauchsfähig ist. In Tabelle 1 wäre eine solche Grenze beispielsweise für ein Δ größer als ±6 zu setzen.

**Tabelle 1**

| Alterungsversuch bei 80% relativer Feuchte mit festem Kontrollbezugswert W1. | | | | | | |
|---|---|---|---|---|---|---|
| t in h | a) W1 in mV | b) L1 in mV | c) Q1 = L1/W1 in % | d) L2 in mV | e) Q2 = L2/W1 in % | f) Δ = Q2-Q1 |
| 0 | 88,5 | 67,6 | 76,4 | - | - | - |
| 48 | - | - | - | 65,8 | 74,4 | -2,0 |
| 96 | - | - | - | 60,3 | 68,1 | -8,3 |
| 144 | - | - | - | 56,7 | 64,1 | -12,3 |
| 192 | - | - | - | 53,4 | 60,3 | -16,1 |

Die Tabelle 2 zeigt einen entsprechenden Alterungsversuch, bei dem im Unterschied zu Tabelle 1 nicht nur am Anfang der Messung, sondern bei jeder zeitlichen Kontrollstufe der Standardreferenzwert W1 neu bestimmt wird. Eine entsprechende Streuung des Standardreferenzwerts W1 kann sich auch dadurch ergeben, daß unterschiedliche Analysegeräte, die sich in ihren optischen und meßtechnischen Eigenschaften unterscheiden, verwendet werden.

Man erkennt in Tabelle 2 aber auch, daß die Streuung des hier zu Versuchszwecken protokollierten Standardreferenzwerts W1 gering ist und dieser nicht dem Alterungsprozeß unterliegt. In der praktischen Anwendung der Erfindung ist es daher nicht erforderlich, die Standardreferenzwerte W1 jedesmal neu zu bestimmen, um anhand der Veränderung der Kontrollwerte L2 eine Alterung zu erkennen.

**Tabelle 2**

| Alterungsversuch bei 80% relativer Feuchte mit geändertem Kontrollbezugswert W1. | | | | | | |
|---|---|---|---|---|---|---|
| t in h | a) W1 in mV | b) L1 in mV | c) Q1 = L1/W1 in % | d) L2 in mV | e) Q2 = L2/W1 in % | f) Δ = Q2-Q1 |
| 0 | 88,5 | 67,6 | 76,4 | - | - | - |
| 48 | 88,1 | - | - | 65,8 | 74,7 | -1,7 |
| 96 | 87,8 | - | - | 60,3 | 68,7 | -7,7 |
| 144 | 88,2 | - | - | 56,7 | 64,3 | -12,1 |
| 192 | 88,3 | - | - | 53,4 | 60,5 | -15,9 |

Die Auswirkung der Alterung auf die Verfälschung des Meßergebnisses für die Glukosekonzentration c ist in Tabelle 3 dargestellt.

**Tabelle 3**

| Glucose-Meßwerte nach Alterung | | | |
|---|---|---|---|
| c für t = 0 in mg/dl | t in h | c (t) in mg/dl | Δ c in % |
| 87,3 | 48 | 86,8 | -0,6 |
| 87,3 | 96 | 79,4 | -9,0 |
| 87,3 | 144 | 74,7 | -14,4 |
| 87,3 | 192 | 71,3 | -18,3 |
| 201,7 | 48 | 207,6 | 2,9 |
| 201,7 | 96 | 194,6 | -3,5 |
| 201,7 | 144 | 183,9 | -8,8 |
| 201,7 | 192 | 172,0 | -14,7 |
| 343,9 | 48 | 350,1 | 1,8 |
| 343,9 | 96 | 332,6 | -3,3 |
| 343,9 | 144 | 313,6 | -8,8 |
| 343,9 | 196 | 299,3 | -13,0 |
| 504,8 | 48 | 510,9 | 1,2 |
| 504,8 | 96 | 474,8 | -5,9 |
| 504,8 | 144 | 446,2 | -11,6 |
| 504,8 | 196 | 422,4 | -16,3 |

Man erkennt, daß ab 96 Std. Belastungszeit t bei 80% relativer Feuchte Abweichungen von mehr als 5% auftreten. Wenn man dementsprechend in den Tabellen 1 und 2 einen Toleranzbereich für die Abweichung Δ von ± 6 wählt, können die gealterten und nicht mehr brauchbaren Testelemente mit dem erfindungsgemäßen Verfahren erkannt und ausgesondert werden.

Das erfindungsgemäße Verfahren bietet erhebliche Vorteile gegenüber dem Stand der Technik, nach dem der Kontrollwert nur gegen einen festen, am Gerät voreingestellten Grenzwert abgeglichen wird. Das Differenzierungspotential ist nach dem Stand der Technik erheblich geringer, so daß Glukosemeßwertabweichungen zwischen unbelasteten und belasteten Mustern erst ab 30% und größer erkannt werden. Die bekannten Verfahren können somit selbst die nach einer Belastungszeit von 192 Stunden auftretende Abweichung des Glukosemeßwerts von ca. 15% (siehe Tabelle 3) nicht erfassen.

Die Erfindung ermöglicht eine einfache und zuverlässige Kontrolle der Gebrauchsfähigkeit von Analyseelementen. Von besonderem Vorteil ist es, wenn die fotometrische Überprüfung des Trockenleerwerts bei jedem Analyseelement benutzerunabhängig, d.h. selbsttätig und automatisch erfolgt, und durch Referenzierung des Meßwertes die Entscheidungsgenauigkeit erheblich gesteigert wird. Um den aktuellen Zustand der Optik des Auswertegeräts zu berücksichtigen und von Schwankungen unabhängig zu werden, wird die aktuelle Geräteoptik durch eine Vergleichsmessung auf einen Weißstandard bezogen. Der Trockenleerwert kontrollierter Analyseelemente wird ebenfalls auf den Weißstandard bezogen, was in Form einer Quotientenbildung erfolgt. Der Kontrollquotient eines kontrollierten Analyseelements wird mit einem ersten Referenzquotienten verglichen. Der erste Referenzquotient wird bei Bedarf aktualisiert, so daß der reale Zustand der Optik des Auswertegeräts und der chargenabhängige Sollwert einer Packung von Analyseelementen jeweils aktuell Berücksichtigung finden. Damit wird ein sehr feines Erkennungsmerkmal für geschädigte Analyseelemente geschaffen.

Zur Überprüfung der Funktionsfähigkeit des erfindungsgemäßen Kontrollverfahrens in einem entsprechenden Auswertegerät können wechselweise geschädigte und nicht geschädigte Analyseelemente in das Auswertegerät eingeführt und die Reaktion des Auswertegeräts überprüft werden. Wenn es korrekt funktioniert, wird es je nach Reihenfolge der zur Überprüfung des Gerätes eingeführten Analyseelemente diese für eine Messung akzeptieren oder zurückweisen sowie ggf. eine neue Messung an einem Referenzfeld durchführen.

### Bezugszeichenliste

- 1: Analyseelement
- 2: Basisschicht
- 3: Testfeld
- 4: Referenzfeld (Referenzkontrollmittel)
- 5: Packung
- 6: Langzeitverpackung
- 7: Vorratsbehältnis
- 8: Kammer
- 9: Öffnung
- 10: Siegelfolie
- L1: Kontrollbezugswert (Trockenleerwert des ersten Analyseelements)
- L2: Kontrollwert (Trockenleerwert des zweiten Analyse-elements)
- Q1: erster Bezugsquotient
- Q2: Kontrollquotient
- W1: erster Standardreferenzwert (erster Weißstandard)
- Δ: Abweichung
- δ: Grenzwert
- t: Zeit
- c: Konzentration

## Patentansprüche

1. Verfahren zum Kontrollieren der Gebrauchstauglichkeit von Analyseelementen (1), bei dem kontrolliert wird, ob ein für mindestens einen Kontrollparameter eines kontrollierten Analyseelements (1) gemessener Kontrollwert (L2) innerhalb eines Toleranzbereichs liegt,
**dadurch gekennzeichnet, daß**
a) in einem ersten Schritt in einer Referenzwertmessung ein erster Standardreferenzwert (W1) an einem Referenzkontrollmittel (4) bestimmt wird, das einen standardisierten Referenzwert für den Kontrollparameter bereitstellt,.
b) in einem zweiten Schritt als Kontrollbezugswert (L1) der Kontrollparameter eines ersten Analyseelements bestimmt wird,
c) in einem dritten Schritt der Quotient aus dem Kontrollbezugswert (L1) und dem ersten Standardreferenzwert (W1) gebildet und für später gemessene Analyseelemente als erster Bezugsquotient (Q1) gesetzt wird,
d) in einem vierten Schritt zur Kontrolle eines zu kontrollierenden zweiten Analyseelements der Kontrollparameter des Analyseelements als Kontrollwert (L2) bestimmt wird,
e) in einem fünften Schritt als Kontrollquotient (Q2) der Quotient aus dem Kontrollwert (L2) und dem ersten Standardreferenzwert (W1) gebildet wird,
f) in einem sechsten Schritt die Abweichung (Δ) zwischen dem Kontrollquotienten (Q2) und dem ersten Bezugsquotienten (Q1) bestimmt wird und
g) das kontrollierte zweite Analyseelement zurückgewiesen wird, wenn die Abweichung (Δ) außerhalb eines vorgegebenen Toleranzbereichs liegt.

2. Verfahren nach Anspruch 1, **dadurch gekennzeichnet, daß** in Schritt g) die Abweichung (Δ) anhand eines relativen Unterschieds zwischen dem Kontrollquotienten (Q2) und dem ersten Bezugsquotienten (Q1) ermittelt wird.

3. Verfahren nach Anspruch 1, **dadurch gekennzeichnet, daß** in Schritt g) die Abweichung (Δ) anhand einer Differenz zwischen dem Kontrollquotienten (Q2) und dem ersten Bezugsquotienten (Q1) ermittelt wird.

4. Verfahren nach Anspruch 1, **dadurch gekennzeichnet, daß** das kontrollierte, zweite Analyseelement in Schritt g) zurückgewiesen wird, wenn der Kontrollquotient (Q2) um mehr als einen vorgegebenen Prozentsatz kleiner als der erste Bezugsquotient (Q1) ist.

5. Verfahren nach Anspruch 1, **dadurch gekennzeichnet, daß** das kontrollierte, zweite Analyseelement in Schritt g) zurückgewiesen wird, wenn der Kontrollquotient (Q2) um mehr als einen vorgegebenen Differenzbetrag kleiner als der erste Bezugsquotient (Q1) ist.

6. Verfahren nach Anspruch 1, **dadurch gekennzeichnet, daß** in Schritt g) ein Toleranzbereich verwendet wird, der chargenspezifisch für die aktuelle Charge der Analyseelemente ist.

7. Verfahren nach einem der vorhergehenden Ansprüche, **dadurch gekennzeichnet, daß** das erste Analyseelement und das zweite, zu kontrollierende Analyseelement Teil einer Packung (5) oder eines Vorratsbehältnisses (7) sind, die weitere gleichartige Analyseelemente enthält und eine den Analyseelementen gemeinsame Langzeitverpackung (6) aufweist.

8. Verfahren nach Anspruch 7, **dadurch gekennzeichnet, daß** die Analyseelemente in der Packung (5) oder dem Vorratsbehältnis (7) jeweils durch ein individuelles Verpackungselement (10) einzeln geschützt sind.

9. Verfahren nach Anspruch 7 oder 8, **dadurch gekennzeichnet, daß** in Schritt b) das Analyseelement (1) gewählt wird, das als erstes einer Packung (5) oder einem in der Packung (5) enthaltenen Vorratsbehältnis (7) entnommen wird.

10. Verfahren nach einem der Ansprüche 7 bis 9, **dadurch gekennzeichnet, daß** die Schritte a) bis c) beim Verwenden einer neuen Packung (5), beim Verwenden eines ersten Analyseelements einer Packung (5) oder eines Vorratsbehältnisses (7) oder beim Öffnen einer Langzeitverpackung (6) durchgeführt werden.

11. Verfahren nach einem der vorhergehenden Ansprüche, **dadurch gekennzeichnet, daß** die Schritte a) bis c) wiederholt werden, wenn an dem die Kontrollmessung bzw. die analytische Messung durchführenden Auswertegerät eine den Meßwert potentiell beeinflussende Änderung durchgeführt wurde.

12. Verfahren nach einem der vorhergehenden Ansprüche, **dadurch gekennzeichnet, daß** beim Feststellen einer vorgegebenen Abweichung in Schritt g) der in Schritt d) bestimmte Kontrollwert als neuer Kontrollbezugswert gemäß Schritt b) verwendet, daraus ein neuer Bezugsquotient gemäß Schritt c) gebildet und der neue Bezugsquotient für die Kontrolle weiterer Analyseelemente gemäß den Schritten d) bis g) zugrunde gelegt wird.

13. Verfahren nach Anspruch 12, **dadurch gekennzeichnet, daß** ein neuer Bezugsquotient gebildet wird, wenn ein in Schritt e) bestimmter Kontrollquotient (Q2) den aktuellen Bezugsquotienten (Q1) um mehr als ein festgelegten Grenzwert (δ) übersteigt.

14. Verfahren nach Anspruch 13, **dadurch gekennzeichnet, daß** ein Grenzwert (δ) verwendet wird, der chargenspezifisch für die aktuelle Charge der Analyseelemente (1) ist.

15. Verfahren nach einem der vorhergehenden Ansprüche, **dadurch gekennzeichnet, daß** einer oder mehrere Verfahrensschritte automatisch durchgeführt werden.

16. Verfahren nach einem der vorhergehenden Ansprüche, **dadurch gekennzeichnet, daß** ein Referenzkontrollmittel (4) verwendet wird, das in Schritt a) einen hohen Meßwert für den Kontrollparameter liefert.

17. Verfahren nach einem der vorhergehenden Ansprüche, **dadurch gekennzeichnet, daß** Analyseelemente verwendet werden, die sowohl ein Kontrollmittel (3) für die Messung des Kontrollbezugswerts (L1) bzw. Kontrollwerts (L2) als auch das Referenzkontrollmittel (4) für die Referenzwertmessung des Schritts a) aufweisen.

18. Verfahren nach einem der vorhergehenden Ansprüche, **dadurch gekennzeichnet, daß** das Analyseelement (1) ein Testelement für einen trägergebundenen Schnelltest zur qualitativen oder quantitativen Analyse von Bestandteilen einer festen oder flüssigen Probe, insbesondere einer Körperflüssigkeit von Menschen oder Tieren, insbesondere zur Bestimmung des Blutglukosegehalts ist.

19. Verfahren nach einem der vorhergehenden Ansprüche, **dadurch gekennzeichnet, daß** der Kontrollparameter ein optischer Meßwert, insbesondere ein Remissionswert ist.

## Claims

1. Method for checking the fitness for purpose of analysis elements (1), in which method it is checked, whether a control value (L2) measured for at least one control parameter of a checked analysis element (1) is within a tolerance range,
**characterized in that**
a) in a first step, a first standard reference value (W1) is determined in a reference value measurement at a reference control means (4) which provides a standardized reference value for the control parameter,
b) in a second step, the control parameter of a first analysis element is determined as control reference value (L1),
c) in a third step, the quotient from the control reference value (L1) and the first standard reference value (W1) is calculated and set as first reference quotient (Q1) for analysis elements measured later,
d) in a fourth step, the control parameter of a second analysis element is determined as control value (L2) for checking the second analysis element to be checked,
e) in a fifth step, the quotient from the control value (L2) and the first standard reference value (W1) is calculated as the control quotient (Q2),
f) in a sixth step, the deviation (Δ) between the control quotient (Q2) and the first reference quotient (Q1) is determined and
g) the checked second analysis element is rejected if the deviation (Δ) is not within a given tolerance range.

2. Method according to claim 1, **characterized in that** in step g) the deviation (Δ) is determined using a relative difference between the control quotient (Q2) and the first reference quotient (Q1).

3. Method according to claim 1, **characterized in that** in step g) the deviation (Δ) is determined using a difference between the control quotient (Q2) and the first reference quotient (Q1).

4. Method according to claim 1, **characterized in that** the checked, second analysis element is rejected in step g), if the control quotient (Q2) is smaller, by a given percentage, than the first reference quotient (Q1).

5. Method according to claim 1, **characterized in that** the checked, second analysis element is rejected in step g), if the control quotient (Q2) is smaller, by a given difference value, than the first reference quotient (Q1).

6. Method according to claim 1, **characterized in that** a tolerance range is used in step g) which is batch-specific for the current batch of analysis elements.

7. Method according to any one of the preceding claims **characterized in that** the first analysis element and the second analysis element which is to be checked, are comprised in a package (5) or in a storage container (7) containing further similar analysis elements and showing a long-time packaging (6) common to all analysis elements.

8. Method according to claim 7, **characterized in that** the analysis elements in the package (5) or in the storage container (7) are individually protected by an individual packaging element (10).

9. Method according to claim 7 or 8, **characterized in that** the analysis element (1) which is the first one to be removed from a package (5) or from a storage container (7) contained in the package (5), is chosen in step b).

10. Method according to any one of claims 7 to 9, **characterized in that** steps a) to c) are performed when a new package (5) is used, when the first analysis element of a package (5) or a storage container (7) is used or when a long-time packaging (6) is opened.

11. Method according to any one of the preceding claims, **characterized in that** the steps a) to c) are repeated, if a change which potentially influences the measured value, was performed on the evaluation device which performs the control measurement or the analytical measurement, respectively.

12. Method according to any one of the preceding claims, **characterized in that** in case of the determination of a given deviation in step g), the control value determined in step d) is used as new control reference value according to step b), a new reference quotient from this is calculated according to step c), and that this new reference quotient is used as a basis for checking further analysis elements according to steps d) to g).

13. Method according to claim 12, **characterized in that** a new reference quotient is formed, if a control quotient (Q2) determined in step e) exceeds the current reference quotient (Q1) by more than a fixed limit value (δ).

14. Method according to claim 13, **characterized in that** a tolerance value (δ) is used, which is batch-specific for the current batch of the analysis elements (1).

15. Method according to any one of the preceding claims, **characterized in that** one or various method steps are performed automatically.

16. Method according to any one of the preceding claims, **characterized in that** a reference control means (4) is used, providing a high measured value for the control parameter in step a).

17. Method according to any one of the preceding claims, **characterized in that** analysis elements are used which comprise a control means (3) for the measurement of the control reference value (L1) or control value (L2), as well as the reference control means (4) for the reference value measurement of step a).

18. Method according to any one of the preceding claims, **characterized in that** the analysis element (1) is a test element for a substrate-based rapid test for the qualitative or quantitative analysis of components of a solid or liquid sample, in particular of body liquids of human beings or animals, in particular for the determination of the blood glucose content.

19. Method according to any one of the preceding claims, **characterized in that** the control parameter is an optical measured value, in particular a remission value.

## Revendications

1. Procédé pour contrôler l'aptitude à l'emploi d'éléments d'analyse (1), dans lequel on contrôle, si une valeur de contrôle (L2) mesurée pour au moins un paramètre de contrôle d'un élément d'analyse contrôlé (1) se trouve dans une plage de tolérances,
**caractérisé en ce que**,
a) dans une première étape dans une mesure de valeur de référence, on détermine une première valeur de référence normalisée (W1) sur un moyen de contrôle de référence (4) qui prépare une valeur de référence normalisée pour le paramètre de contrôle,
b) dans une deuxième étape, on détermine un premier élément d'analyse en tant que valeur'de référence de contrôle (L1) du paramètre de contrôle,
c) dans une troisième étape, on forme le quotient de la valeur de référence de contrôle (L1) et de la première valeur de référence normalisée (W1) et on le fixe en tant que premier quotient de référence (Q1) pour des éléments d'analyse mesurés ultérieurement,
d) dans une quatrième étape pour le contrôle d'un deuxième élément d'analyse à contrôler, on détermine le paramètre de contrôle de l'élément d'analyse en tant que valeur de contrôle (L2),
e) dans une cinquième étape, on forme le quotient de la valeur de contrôle (L2) et de la première valeur de référence normalisée (W1), en tant que quotient de contrôle (Q2),
f) dans une sixième étape, on détermine l'écart (Δ) entre le quotient de contrôle (Q2) et le premier quotient de référence (Q1) et
g) le deuxième élément d'analyse contrôlé est refusé lorsque l'écart (Δ) se trouve en dehors d'une plage de tolérance prédéterminée.

2. Procédé selon la revendication 1, **caractérisé en ce que** dans l'étape g), on détermine l'écart (Δ) à l'aide d'une différence relative entre le quotient de contrôle Q2 et le premier quotient de référence (Q1).

3. Procédé selon la revendication 1, **caractérisé en ce que** dans l'étape g), on détermine l'écart (Δ) à l'aide d'une différence entre le quotient de contrôle (Q2) et le premier quotient de différence (Q1).

4. Procédé selon la revendication 1, **caractérisé en ce que** l'on refuse le deuxième élément d'analyse contrôlé dans l'étape g), lorsque le quotient de contrôle (Q2) est inférieur au premier quotient de référence (Q1) de plus d'un pourcentage prédéterminé.

5. Procédé selon la revendication 1, **caractérisé en ce que** l'on refuse le deuxième élément d'analyse contrôlé dans l'étape g), lorsque le quotient de contrôle (Q2) est inférieur au premier quotient de référence (Q1) de plus d'une différence prédéterminée.

6. Procédé selon la revendication 1, **caractérisé en ce que** dans l'étape g), on emploie une plage de tolérance qui est spécifique à la charge pour la charge actuelle des éléments d'analyse.

7. Procédé selon l'une quelconque des revendications précédentes, **caractérisé en ce que** le premier élément d'analyse et le deuxiéme élément d'analyse à contrôler font partie d'un conditionnement (5) ou d'un récipient de réserve (7) qui contient d'autres éléments d'analyse du même type et présente un conditionnement de longue durée (6) commun aux éléments d'analyse.

8. Procédé selon la revendication 7, **caractérisé en ce que** les éléments d'analyse dans le conditionnement (5) ou le récipient de réserve (7) sont protégés à chaque fois individuellement par un élément de conditionnement individuel (10).

9. Procédé selon la revendication 7 ou 8, **caractérisé en ce que** dans l'étape b), on choisit l'élément d'analyse (1) qui est retiré le premier d'un conditionnement (5) ou d'un récipient de réserve (7) contenu dans le conditionnement (5).

10. Procédé selon l'une quelconque des revendications 7 à 9, **caractérisé en ce que** l'on réalise les étapes a) jusqu'à c), lorsqu'on emploie un nouveau conditionnement (5), lorsqu'on emploie un premier élément d'analyse d'un conditionnement (5) ou d'un récipient de réserve (7) ou lorsqu'on ouvre un conditionnement à longue durée (6).

11. Procédé selon l'une quelconque des revendications précédentes, **caractérisé en ce que** l'on répète les étapes a) à c) lorsqu'on a réalisé une modification influençant potentiellement la valeur de mesure sur l'appareil d'évaluation qui réalise la mesure de contrôle resp. la mesure analytique.

12. Procédé selon l'une quelconque des revendications précédentes, **caractérisé en ce que** lorsqu'on constate un écart prédéterminé dans l'étape g) on emploie la valeur de contrôle déterminée dans l'étape d) comme nouvelle valeur de référence de contrôle selon l'étape b), on forme un nouveau quotient de référence à partir de cela selon l'étape c) et le nouveau quotient de référence sert de base pour le contrôle d'autres éléments d'analyse selon les étapes d) à g).

13. Procédé selon la revendication 12, **caractérisé en ce que** l'on forme un nouveau quotient de référence lorsqu'un quotient de contrôle déterminé dans l'étape e) (Q2) dépasse le quotient de référence présent (Q1) de plus d'une valeur limite fixée (δ).

14. Procédé selon la revendication 13, **caractérisé en ce que** l'on emploie une valeur limite (δ), qui est spécifique à la charge pour la charge actuelle des éléments d'analyse (1).

15. Procédé selon l'une quelconque des revendications précédentes, **caractérisé en ce qu'**une ou plusieurs étapes de procédé sont réalisées automatiquement.

16. Procédé selon l'une quelconque des revendications précédentes, **caractérisé en ce que** l'on emploie un moyen de contrôle de référence (4), qui livre dans l'étape a) une valeur de mesure élevée pour le paramètre de contrôle.

17. Procédé selon l'une quelconque des revendications précédentes, **caractérisé en ce que** l'on emploie des éléments d'analyse qui présentent aussi bien un moyen de contrôle (3) pour la mesure de la valeur de référence de contrôle (L1) resp. la valeur de contrôle (L2) que le moyen de contrôle de référence (4) pour la mesure de la valeur de référence de l'étape a).

18. Procédé selon l'une quelconque des revendications précédentes, **caractérisé en ce que** l'élément d'analyse (1) est un élément de test pour un test rapide lié au support pour une analyse qualitative ou quantitative de composants d'un échantillon solide ou liquide, en particulier, d'un liquide corporel d'humains, ou d'animaux, en particulier pour déterminer la glycémie sanguine.

19. Procédé selon l'une quelconque des revendications précédentes, **caractérisé en ce que** le paramètre de contrôle est une valeur de mesure optique, en particulier une valeur de réflectance.
